(19) **Europäisches Patentamt**
**European Patent Office**
**Office européen des brevets**

(11) **EP 2 874 614 B1**

(12) **EUROPEAN PATENT SPECIFICATION**

(45) Date of publication and mention
of the grant of the patent:
**23.08.2017 Bulletin 2017/34**

(21) Application number: **13770508.3**

(22) Date of filing: **15.07.2013**

(51) Int Cl.:
*A61K 9/70* (2006.01)     *A61K 9/00* (2006.01)
*A61K 38/39* (2006.01)    *A61L 15/28* (2006.01)
*A61L 15/32* (2006.01)    *A61L 15/44* (2006.01)
*A61L 15/22* (2006.01)    *A61L 26/00* (2006.01)

(86) International application number:
**PCT/IB2013/055807**

(87) International publication number:
**WO 2014/013413 (23.01.2014 Gazette 2014/04)**

(54) **HYDROSOLUBLE FILM HAVING HEALING ACTIVITY**

WASSERLÖSLICHE FOLIE MIT HEILENDER WIRKUNG

FILM HYDROSOLUBLE PRÉSENTANT UNE ACTIVITÉ DE CICATRISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.07.2012 IT MI20121248**

(43) Date of publication of application:
**27.05.2015 Bulletin 2015/22**

(73) Proprietor: **Mediolanum Farmaceutici S.p.A.
20143 Milano (IT)**

(72) Inventors:
• **DEL BONO, Alessandro
I-20143 Milan (IT)**
• **DEL BONO, Cristina
I-20143 Milan (IT)**
• **FERRARI, Gianni
I-20143 Milan (IT)**

(74) Representative: **Asensio, Raffaella Consuelo et al
Perani & Partners S.p.A.
Piazza San Babila, 5
20122 Milano (IT)**

(56) References cited:
**WO-A2-03/034993     US-A1- 2010 029 790**

• **KIRKER K R ET AL: "Glycosaminoglycan
hydrogel films as bio-interactive dressings for
wound healing", BIOMATERIALS, ELSEVIER
SCIENCE PUBLISHERS BV., BARKING, GB, vol.
23, no. 17, 1 September 2002 (2002-09-01), pages
3661-3671, XP027303699, ISSN: 0142-9612
[retrieved on 2002-09-01]**
• **AROSIO E ET AL: "A placebo-controlled,
double-blind study of mesoglycan in the
treatment of chronic venous ulcers.",
EUROPEAN JOURNAL OF VASCULAR AND
ENDOVASCULAR SURGERY : THE OFFICIAL
JOURNAL OF THE EUROPEAN SOCIETY FOR
VASCULAR SURGERY OCT 2001, vol. 22, no. 4,
October 2001 (2001-10), pages 365-372,
XP002693997, ISSN: 1078-5884**
• **LARS ALEXANDER SCHNEIDER ET AL:
"Influence of pH on wound-healing: a new
perspective for wound-therapy?", ARCHIVES OF
DERMATOLOGICAL RESEARCH ; FOUNDED IN
1869 AS ARCHIV FÜR DERMATOLOGIE UND
SYPHILIS, SPRINGER, BERLIN, DE, vol. 298, no.
9, 8 November 2006 (2006-11-08), pages 413-420,
XP019473786, ISSN: 1432-069X cited in the
application**

Remarks:
The file contains technical information submitted after
the application was filed and not included in this
specification

EP 2 874 614 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention concerns a water-soluble film and the device for medical use containing said water-soluble film with healing activity, in particular suitable as adjuvant in the systemic pharmacological therapy of sores or wounds.

STATE OF THE ART

Proteoglycans and glycosaminoglycans are components of the extracellular matrix and of the cellular surface.

In particular, proteoglycans consist of a nucleoprotein and one or more chains of glycosaminoglycans. Glycosaminoglycans (GAGs) are linear polysaccharides with two repetitive disaccharide units consisting, respectively, of two families of amino sugars: glucosamine sulphate (GlcNS, GlcNS,6S, GlcN,6S) glucosamine acetate (GlcNAc, GlcNAc,6S) and galactosaminoacetate (GalNAc, GalNAc,4S; GalNAc,6S; GalNAc,4S,6S) and of a uronic acid selected from glucuronic acid (GlcA) and iduronic acid (IdoA, IdoA2S).

Virtually all of the cells of mammals produce proteoglycans and/or excrete them in the extracellular matrix, insert them in the plasma membrane or store them in the secretory granules.

Following to a wound or lesion of the skin, many stimulation mechanisms (induction) of proteoglycans are activated according to a specific cellular behaviour. For example, sindecan (proteoglycan of dermatan sulphate) increases many times the concentration thereof both in the endothelial cells and in the hyperproliferating keratinocytes (Elnius K. et al., J. Cell. Biol. 1991; 114: 585-595. Gallo et al. J. Invest. Dermatol. 1996; 107: 676-683.

The analysis of soluble proteoglycans, extracted from the exudate of wounds and/or lesions, shows that the galactosaminoglycan sulphate is present in greater quantities is dermatan sulphate (DS). Dermatan sulphate is a strong promoter of fibroblast growth factors (FGF-2) (Penc et al., J. Biol. Chem. 1998; 273(43) : 28116-28121). Human endothelial cells in culture, exposed to dermatan sulphate solutions, responded with the production of NF-KB and increased the cellular adhesion of type 1 (ICAM-1) of endothelial leukocytes, whereas this protein contributed to the repairing action of the lesions (Penc SF et al., J. Clin. Invest. 1999; 103(9): 1329-1335).

DSPGs, i.e. dermatan sulphate proteoglycans, such as decorin and biglycan, are strong promoters of growth factors FGF2 and have been associated with the organisation and the growth of scarring of the wounds (Garg et al. "Chemistry of Scarring" in "Scarless Wound Repair" Marcel Dekker New York 2000, pages 1-2.).

Chronic wounds and sores contain a high concentration of proteases and lower concentrations of growth factors, high presence of secretory cells with low mytogenic activity and high concentrations of inflammatory cytokines. The proteases cause the degradation of the endogenous growth factors, of the fibronectine and laminine, preventing or slowing down the spontaneous regression of lesions (Traversa B. et al. Primary Intention 2001 9(4): 161-167).

The next study was that of checking whether exogenous glycosaminoglycans thus no longer in the form of proteoglycans could perform a decisive role in the treatment of such chronic sores and wounds.

It was demonstrated, as a result of experiments carried out *in vitro* in collagen matrices designed to stimulate tissue engineering, that fibroblasts and keratinocytes displayed a greater proliferation capability on heparin/collagen or dermatan sulphate/collagen matrices if compared to the sole collagen matrix. This effect was increasedly marked the greater the molecular weight of the GAGs. The presence of dermatan sulphate in the membranes of collagen promoted the attraction, the growth and the proliferation of fibroblasts and keratinocytes, whereas the presence of unfractioned heparin mainly promoted the proliferation of fibroblasts (Ruozi B. et al., Int J. Pharmaceutics 2009 ; 738 (1-2) :108-115). However, for all devices for topical use described in the state of the art it seems essential to associate the aforementioned GAGs with the collagen, to obtain appreciable results.

US 4837024 describes collagen powders and at least one glycosaminoglycan to be applied on wounds.

This prior art document describes the ability of various GAGs to attract fibroblasts and promote neovascularization. Indeed, both activities are important parameters for evaluating the ability of GAGs in promoting wound healing. The ability of GAGs in both activities cannot work without the presence of collagen.

Also for US 5,929,050 that describes a composition for treating wounds consisting of an aqueous solution containing chondroitin sulphate and of a sterile bandage, the presence of collagen seems important to speed up healing since the latter provides all the nutritional substances for cellular growth.

Kirker et al in "Glycosamin hydrogel films as biointeractive dressings for wound healing"-Biomaterials Elsevier Science Publisher BV Barking GB, Vol.23 No.17 1 September 2002 pages 3661-3671 disclose a film in which a glycosaminoglycan as hyaluronic acid or chondroitin sulphate are an integral part of the structure of the film since they form covalent chemical bonds with the polyethylene glycol by means of the carboxyl groups of the glycosaminoglycan that first are reacted with adipic acid dihydrazide before reacting with polyethylene glycol.

This film does not have sufficient properties of compactness and strength since it is applied on a bandage to protect the wound from bacterial infections. Arosio et al (EUROPEAN JOURNAL OF VASCULAR AND ENDOVASCULAR SURGERY : THE OFFICIAL JOURNAL OF THE EUROPEAN SOCIETY FOR VASCULAR SURGERY OCT 2001, vol. 22, no. 4, October 2001 (2001-10), pages 365-372, XP002693997, ISSN: 1078-5884) describes mesoglycan in the treatment of chronic venous ulcers. Finally, we quote WO03034993 that describes some compositions to promote cellular growth to protect the tissues and to promote the healing of wound based on

a) hydrolysed collagen, as main component,
b) a GAG polysulphate.
c) a hyaluronic acid salt
d) a glucosamine salt

These compositions can also be in film form, in this case the collagen has the function of the main filming substance.

As known, collagen available on the market is essentially of bovine and equine origin; in some individuals it can induce an antigenic response, a response that can be substantially reduced if in the extraction and purification processes of collagen suitable proteolithic enzymes are used that are suitable for destroying its C-terminal telopeptide.

Moreover, collagen is a very expensive substance and therefore not recommended due to the resulting price, if used in large quantities like for example those necessary for preparing films suitable for covering large dimension wounds. As a demonstration thereof the aforementioned WO03034993 encompasses the use of such types of film only for smaller size wounds such as the dental ones.

The need is therefore felt to have a device for scar formation even of large lesions like for example ulcers from vasculopathies and diabetic foot that at the same time protects the wound and continuously and uniformly releases the GAGs *in situ* in the lesion without the drawbacks of the state of the art.

US2010/002790 describes water-soluble films containing

a) between 25 and 60% by weight of sodium alginate
b) between 0.1 and 20% by weight of microcrystalline cellulose over the total weight of the film,
c) between 0.1 and 25% by weight of vegetable proteins.

When used for topical use on the skin these films can be used for treating small wounds of the skin and they do not contain active ingredients having healing activity.

SUMMARY OF THE INVENTION

The Applicant has now unexpectedly found that it is possible to overcome the drawbacks of devices for topical use containing GAGs with the device of the present invention comprising a water-soluble film or solid gel having healing activity according to the present invention.

Therefore, the present invention relates to a water-soluble film comprising:

a) between 25 and 60% by weight of sodium alginate, as main filmogenous substance,
b) between 0.1 and 20% by weight of microcrystalline cellulose over the total weight of the film,
c) between 0.1 and 25% by weight of vegetable proteins ,
d) at least one glycosaminoglycan sulphate of the natural type, as active ingredient with healing activity.

The present invention further relates to a device for medical use containing the water-soluble film according to the present invention.

This device is in particular suitable for covering sores and wounds, even large ones, protecting them from the external environment, continuously providing the glycosaminoglycan sulphate of the natural type in the area of the damaged skin, since it performs a slow release action of the active ingredient in the required area and at the same time protects the site of the lesion from bacterial agents, pollutants, foreign bodies.

DESCRIPTION OF THE FIGURES

Figure 1 describes the buffering action of the biofilm, after addition of HCl or NaOH as shown in the experiment described in example 2 compared with pure water and aqueous solutions containing 30 mg of Mesoglycan.

Figure 2 shows the progression of the pH of the film of the present invention prepared as described in example 5, placed in solutions made acidic with 0.01 M HCl or basic with 0.01 M NaOH, and the comparison of the pH values in pure water and in aqueous solutions containing 30 mg of Mesoglycan, after the additions of the corresponding volumes of NaOH and HCl.

DETAILED DESCRIPTION OF THE PRESENT INVENTION

For the purposes of the present invention by the general definition of "glycosaminoglycan sulphates and/or galactosaminoglycan sulphates (GAGs)" we mean both glycosaminoglycan sulphates and/or galactosaminoglycan sulphates that are found in nature for example in animal tissues and in particular in the extracellular matrix as components of the so-called proteoglycans like for example chondroitin sulphate, dermatan sulphate, heparan sulphate, heparin with low, relatively low and/or high molecular weight, cheratan sulphate and relative mixtures of at least two of the aforementioned components.

For the purposes of the present invention by heparin with low molecular weight we mean heparin with average weight molecular weight (Mw) comprised between: 4000 and 6500 and with 60% of the molecules with average molecular weight of less than 8000 Da.

By heparin with relatively low molecular weight we mean: heparin with average molecular weight (Mw) comprised between: 6500 and 10500 and with at least 60% of the molecules characterised by an average molecular weight of less than 12000 Da.

By heparin with high molecular weight we mean conventional unfractionated heparin (UFH) having an average molecular weight comprised between: 12000 and 18000 and even reaching values up to 40000 Da

For the purposes of the present invention by the definition "chondroitin sulphate", where not expressly specified we mean at least one of the following isomers: chondroitin-4-sulphate (Ch-S-A), chondroitin-6-sulphate(Ch-S-C), chondroitin-2,6-di-sulphate(Ch-S-D), chondroitin-4,6-disulphate (Ch-S-E)

According to a preferred embodiment, the medical devices according to the present invention contain a mixture of at least 2 out of the aforementioned galactosaminoglycan sulphates of the natural type.

According to a preferred embodiment of the invention these medical devices preferably contain the following mixtures:

Mixture (A), mainly comprising:

α) heparin with relatively low molecular weight, with possible traces of heparin containing glucosamine N-desulphate,(GlcN, GlcN,6S)
β) dermatan sulphate
γ) chondroitin sulphate;

Mixture (B), comprising

α') heparin with relatively low molecular weight,
β') dermatan sulphate,

Mixture (C) comprising the following glycosaminoglycans with low molecular weight:

α") heparan sulphate,
β") chondroitin sulphate
γ") dermatan sulphate

Mixture (D) comprises the following components:

α''') dermatan sulphate with traces of heparin with relatively low molecular weight and
β''') chondroitin sulphate,

The family of mixture (A) includes in particular the product available on the market with the trade name Mesoglycan®. Preferably, the Mesoglycan mixture consists of:

- 55-60% of a mixture of heparin with relatively low molecular weight of whose 2-8% is heparin with high molecular weight (slow moving heparin) and 47-58% heparin (so-called fast moving heparin).
- 25-35% of dermatan sulphate,

- 4-8% of chondroitin sulphate (mainly Ch-S-A and smaller quantities of Ch-S-C.)

The family of mixtures (B) includes in particular the product known as Sulodexide containing 80% heparin with relatively low molecular weight and 20% dermatan sulphate.

The family (C) includes in particular the product better known as Danaparoid consisting of 75-85% heparan sulphate, no more than 8,5% by weight chondroitin sulphates (Ch-S-A) and (Ch-S-C), and 8 to 16% dermatan sulphate.

The family (C) also includes the product known as Mistral.

The water-soluble film according to the present invention is preferably prepared as described in US2010/002790.

For the purposes of the present invention by dry weight of the water-soluble film we mean the weight of the components in the final film other than water.

For the purposes of the present invention, as main filming substance we consider the substance that represents between 90 and 100% by weight of filming substance over the total weight of the filming substances, preferably between 95 and 99.9% by weight over the total weight. Preferably, the device according to the present invention contains sodium Mesoglycan, at concentrations comprised between 0.1 and 10% by weight, more preferably between 1 and 7% by weight and even more preferably between 3 and 6% by weight over the dry weight of the water-soluble film. According to a particularly preferred solution it is contained in a quantity of 4.5 and 4.8% by weight calculated over the dry weight of the water-soluble film.

The device according to the present invention may also contain dermatan sulphate with traces of heparin with relatively low molecular weight and chondroitin sulphates, at concentrations comprised between 0.1 and 10% by weight, more preferably between 1 and 7% by weight and even more preferably between 3 and 6% by weight over the dry weight of the water-soluble film. According to a particularly preferred solution it is contained in a quantity of 4.5 and 4.8% by weight calculated over the dry weight of the water-soluble film.

Preferably, sodium alginate or component (a) of the water-soluble film, with film-forming properties according to the present invention is contained in quantities comprised between 28 and 40% by weight, more preferably between 30 and 38% by weight and even more preferably between 35 and 37% and according to a particularly preferred solution between 36.1 and 36.9 % by weight calculated over the total dry weight of the water-soluble film.

Microcrystalline cellulose or component (b) used as thickener in the water-soluble film of the invention is contained in quantities preferably comprised between 2.5 and 11% by weight and more preferably comprised between 5 and 8% by weight calculated over the total dry weight of the film.

Vegetable proteins or component (c) of the water-soluble film of the present invention that are contained in the water-soluble film for their emulsifying properties and ability to increase the consistency thereof are present in quantities comprised between 5 and 15%, even more preferably between 10 and 14 % by weight over the total dry weight of the film.

The film may optionally also contain other additives such as polyoxyethylene derivatives and in particular polysorbate 80, preferably in concentrations comprised between 5 and 12%, plasticizers and humectants such as polyols and in particular glycerine preferably in concentrations comprised between 15 and 30%, further thickening agents such as polyvinylpyrrolidone at concentrations preferably comprised between 1 and 5% by weight over the dry weight of the film.

As optional filmogenous or film-forming substance it is possible to use hyaluronic acid in any case in quantities not greater than 0.2% by weight over the total dry weight of the polymer.

The film contains residual humidity, which in any case does not exceed 15% by weight over the total weight of the water-soluble film and in such a way that the activity of the water Aw taken as the ratio between the tension of vapour in the film and the tension of vapour in the pure water, at the same temperature, is comprised between 0.1 and 0.6.

The Applicant has in particular found that the water-soluble film according to the present invention has a higher cicatrizing rate of wounds than the control already after 2 and 4 hours after administration.

Moreover, the Applicant has unexpectedly found that the biomedical device subjected to sterilization through gamma rays is stable, does not change and also has a buffering action, as demonstrated by the *in vitro* experiments given in examples 2 and 5. In fact, the matrix of the water-soluble film can be prepared so as to ensure a final pH, i.e. when the film is in the presence of aqueous solution, comprised in the ranges $6.0 \pm 0.4$ up to pH $7.0 \pm 0.5$ is maintained in this range both by addition of hydrochloric acid and of sodium hydroxide at concentrations 0.01 N and with volumes comprised between 20 $\mu$L and 1000$\mu$L, to the system consisting of an entire membrane suspended in 20 mL of distilled water.

From the prior art the following information can be obtained.

The pH of the intact skin is about 5.5; the pH of most cells is comprised between 6.5 and 8.5; wounds and lesions also have this pH range, with the tendency to reach the value 9.0. The pH of venous blood is 7.35.

In the presence of chronic lesions the pH of the wound bed is of vital importance for the purposes of the healing process; in fact it has been observed that an "acidification" of the bed of the lesion leads to an increase in the healing rate in patients suffering from venous ulcers of the lower limbs.

One of the reasons is the increased availability of oxygen for the tissues. The other derives from the high levels of proteases associated with alkaline pHs of the bed of the lesion. The excessive expression of proteases is a factor that contributes to the slowing down of the healing process, in some cases blocking the lesion in a chronic cycle. The reduction of the pH or keeping the fluids of the lesion in a tendentially "acidic" zone, on the other hand, is a method for controlling the activity of the proteases, without furthermore causing an irreversible deactivation thereof, which would determine the denaturing also of useful proteins in the fluids of the wound, like growth factors.

There is evidence that tendentially acidic values of the exudate of the lesion can promote healing. In fact, in tendentially alkaline environments with pH comprised between 7.0 and 9.0 proteases show the peak of maximum activity. Catepsin G, elastase, plasmin, matrix metal-proteinases (MMP-1) can degrade the tissue of the cellular matrix that has just been built (laminin, fibronectin, collagen etc.) and thus slow down healing. (Greener B et al. J of Wound Care 2005; 14 (2): 59 - 61. See also: Rushton I. Role of proteases and of pH in wound healing. Nurs Stand 2007; 21(32): 68, 70, 72. PubMed).

Chronic ulcers and lesions are characterised by pH values even above 9.

There is thus a need to have a medical device capable of inhibiting the alkalinity of lesion and keeping its pH around values of 6.7 - 6.9.

A study of 50 patients carrying acute and chronic lesions was examined in a university hospital (Varanasi - India). The pH values were monitored, detected in the exudate of the wound: at start over 50% of cases had a pH of over 9.0 and no case has a pH of less than 7.5. During the evolution towards healing the pH values fell below 7.5 (14% of cases) and others remained between 7.5 - 8.0 (34% of cases). The evolution of the pH towards values close to neutral was an indication of healing, and furthermore exudates with pH values of over 8.0 represented a good growth medium for *Pseudomonas aeruginosa, Escherichia coli, Klebsiella pneumoniae, Proteus* (V.K. Shukla et al. J of Wound Care 2007; 16 (7): 291-294. PubMed)

Ian A.I. Wilson et al. (in VASA 1979;8 (4): 339 - 342 through: www.scirus.com) in the report titled *"Relazione fra il pH della superficie delle ulcere varicose and guarigione"* shows the results of an experiment on 29 patients suffering from varicose ulcers. The average pH of the exudates of the ulcers was about $7.7 \pm 0.3$. A therapeutic treatment was carried out with ointment buffered at pH 6.0 against a control consisting of the same ointment at pH 7.3. The treatment with ointment buffered to pH 6.0 kept the "acidity" of the venous ulcers for a longer period with a significant advantage in the healing rate measured through a "healing quotient" represented by the area of the part covered by the epithelium, in $mm^2$/day. The values reported were 22.6 $mm^2$/day for those treated and 3.3 $mm^2$/day for the controls.

Alexander L et al. (in the monograph reported in Arch Dermatol Res 2007; 298: 413-420. PubMed) hypothesizes the conditions influencing the pH in the healing of lesions as a new prospect of therapy.

Even better results in terms of the buffering activity of the water-soluble film object of the present invention are obtained if an organic hydroxy acid is added as component of the preparation mixture of the film. According to a preferred embodiment an organic hydroxy acid with from 3 to 6 carbon atoms and containing from 1 to 2 hydroxyl groups is used, like for example: citric acid, tartaric acid, tartronic acid, hydroxybutyric acid, etc..

Preferably citric acid is used.

This buffering characteristic of the film was obtained through addition of a known amount (preferably comprised between 1 and 2 % over the total weight of the dry film) of hydroxy acid to the preparation mixture until a pH comprised between 5.4 and 5.6 is reached.

In practice, by changing this addition, it is possible to give the matrix of the biofilm the ability to keep lesions and sores "buffered" in the pH environments suitable for accelerating their healing.

A further property shown by the water-soluble films of the invention is the high stability in storage.

The device for medical use consisting of the film according to the present invention is in particular suitable for the treatment of secondary lesions to pathological events, like for example ulcers of the lower limbs in subjects with chronic venous insufficiency, diabetic foot ulcer, arterial ulcers, secondary lesions to traumatic events like for example bedsores, abrasions, secondary lesions to environmental events like for example lesions from scalding, bums, all of these lesion also being large.

For illustrating but not limiting purposes we give some examples of composition of the device for medical use consisting of the water-soluble film according to the present invention.

## EXAMPLE 1

### Preparation of the mesoglycan biofilm

In a mixer containing distilled water as need to make up 100 parts, preheated to between 50°C and 70°C the following are dispersed: sorbitol 2.5 parts, glycerine 5 parts, polysorbate 80 parts 2 until complete solubilization. The following are added by sprinkling: sodium alginate 8.7 parts, microcrystalline cellulose 1.7 parts, vegetable protein 3 parts, polyvinylpyrrolidone 0.5 parts, sodium Mesoglycan 1.1 parts, sodium hyaluronate 0.025 parts. The mixture is slowly stirred for 30 minutes until complete dispersion and homogenisation of the gel. A slow cooling down to 25°C begins under slow agitation. The pH of the gelatinous mass is checked, which must be $7.0 \pm 0.5$; in this case it was pH 7.0.

The mass is left to rest for one night for de-aeration, then it is transferred, under stirring and through a peristaltic pump into a suitable container of the spreading machine and then layered in film on a stripe support of polyethylene siliconate. The stripe is passed into a ventilated tunnel oven equipped with four successive heating stations with increasing temperatures up to 120°C. At the exit from the tunnel a reel is obtained consisting of support of polyoxyethylene terephthalate (PET) siliconate and a solid film of gel. The product is seasoned for at least 24 hours. The reel is cut into smaller-banded reels in relation to the desired piece size.

A water-soluble film of 8 x 12 cm is thus obtained weighing on average 650 mg and having the composition expressed over the dry substance as reported in the following table

| Ingredients trade name | Ingredients Chemical name | Composition mg/film |
|---|---|---|
| Protanal GP 2650 | Sodium alginate | 237.86 |
| Avicel PH 105 | Microcrystalline cellulose | 46.48 |
| Pisane M9 | Vegetable protein (peas) | 82.01 |
| Sorbitolo 70% | Sorbitol | 47.84 |
| Pricerina 9095 | Glycerine 99,95% | 136.70 |
| Tween 80 | Polysorbate 80 | 54.68 |
| Kollidon 90 F | Polyvinylpyrrolidone | 13.67 |
| Mesoglycan sodico | sodium Mesoglycan | 30.10 |
| Crystal Hyal | Sodium hyaluronate | 0.68 |

The film has a water content, measured with the Karl Fischer technique of 12% ÷ 15%.

## EXAMPLE 2

### Tests of the buffering effect of the water-soluble film of the invention.

The water-soluble film prepared as described in example 1 was crushed into tiny pieces and it was suspended in 20 mL of distilled water, under agitation. After 5 - 10 minutes, a "solution" formed with a modest amount of material in suspension. The pH of such a system was on average 6.75. At the same time two beakers were prepared containing 20 mL of distilled water, in each of which the pH value was measured and to which, 0.1, 0.2, 0.4, 1.0 mL of sodium hydroxide or hydrochloric acid 0.01 M, respectively, were progressively added separately. In parallel to this test on distilled water the same additions were made, separately, on two suspensions of the same number of membranes of film in 20 mL of distilled water. For each addition the pH values were measured.

The results are given in the following tables 1a and 1b:

Table 1a

| | pH biofilm (*) | | | |
|---|---|---|---|---|
| NaOH 0.01N added (μL) | Lot. P1251A0 | Lot. P1270C0 | Lot. P1271C0 | pH H$_2$O(**) (20 mL) |
| _ | 6.7 | 6.7 | 6.7 | N.D. |
| 20 | 6.8 | 6.7 | 6.8 | 6.6 |
| 100 | 6.8 | 6.8 | 6.8 | 8.6 |
| 200 | 6.9 | 6.8 | 6.9 | 9.1 |
| 400 | 7.0 | 7.0 | 7.1 | 9.5 |
| 1000 | 7.5 | 7.4 | 7.7 | 10.0 |

Table 1b

| HCl 0.01 added (μL) | pH biofilm (*) | | | pH H₂O(**) (20 mL) |
|---|---|---|---|---|
| | Lot. P1251A0 | Lot. P1270C0 | Lot. P1271C0 | |
| _ | 6.9 | 6.8 | 6.8 | N.D. |
| 20 | 6.8 | 6.8 | 6.8 | 6.2 |
| 100 | 6.8 | 6.7 | 6.7 | 4.6 |
| 200 | 6.7 | 6.6 | 6.6 | 4.2 |
| 400 | 6.6 | 6.5 | 6.5 | 3.8 |
| 1000 | 6.3 | 6.2 | 6.2 | 3.4 |

(*) biofilm dissolved in 20 mL of $H_2O$
(**) average of three measurements
N.D. = not able to be determined (after 30 minutes the reading was not yet stable) From this experimental model it was found that the film dissolved/suspended in water has a pH of about 6.75. Its buffering effect would allow the pH of the "lesion system" to be kept around this value or at most comprised between 6.1 and 7.5; as has been demonstrated from those systems, aqueous systems not previously buffered, would reach pH 3.4 and 10.0 respectively.

**Comparative test carried out with Mesoglycan in water.**

An amount of Mesoglycan corresponding to the amount contained in a membrane, 30 mg, was dissolved in 20 mL of distilled water. The pH was measured. Such a solution was added with 20, 100, 200, 400, 1000 μL of NaOH 0.01 M and the pH of the respective solutions was measured. A similar Mesoglycan solution in 20 mL of water was added with corresponding volumes of HCl 0.01 M and the respective pHs were detected. The values obtained are given in Table 1c.

Table 1 c

| NaOH 0.01M added (μL) | pH of 30 mg of Mesoglycan in 20 mL of solutions made basic (with NaOH 0.01 M) and acidic (with HCl 0.01M) through addition of the amounts indicated at the side. | | | HCl 0.01 M added (μL) |
|---|---|---|---|---|
| | Mesoglycan In 20 mL of water + NaOH 0.01 M | | Mesoglycan in 20 mL of water + HCl 0.01 M | |
| _ | 7.5 | | 7.6 | - |
| 20 | 7.6 | | 7.3 | 20 |
| 100 | 9.0 | | 6.8 | 100 |
| 200 | 9.6 | | 6.4 | 200 |
| 400 | 10.2 | | 5.9 | 400 |
| 1000 | 10.6 | | 5.3 | 1000 |

The values of Table 1c demonstrate that the sole solution of Mesoglycan is not able to buffer the zone of the lesion.

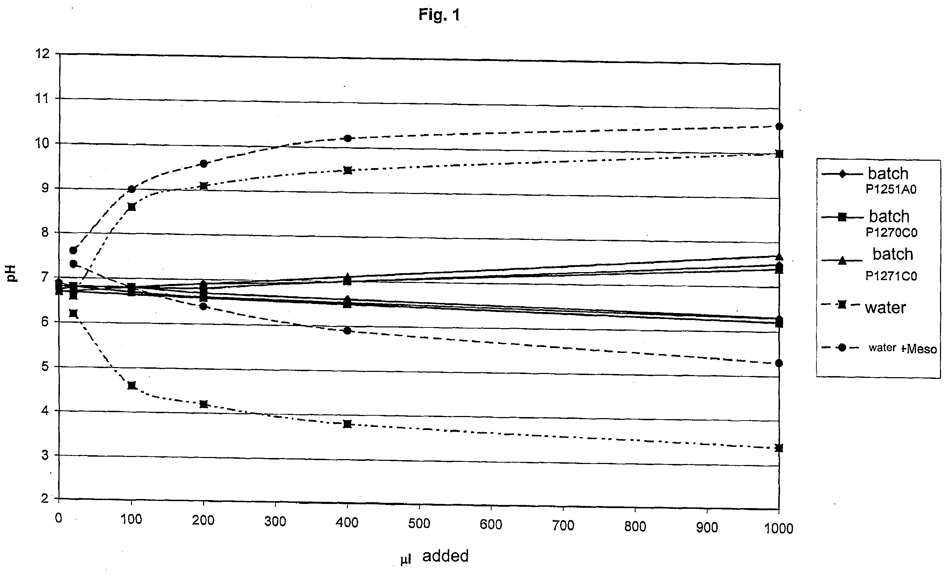

Figure 1 summarises the results obtained with the experiment of example 2, from which it can clearly be seen also graphically that the buffering action is obtained only with the water-soluble films according to the present invention.

Fig. 1 shows the pH values taken by the three samples P1251A0 ♦, P1270C0 ■, P1271C0 ▲ in comparison with aqueous solutions ■, and solutions of 30 mg of Mesoglycan in 20 mL of water ●, after each addition of the respective amounts of NaOH and HCl 0.01M.

## EXAMPLE 3

### Stability study

The purpose of this experiment was to evaluate the Mesoglycan content over time by means of its activated anti-Xa factor activity.

### 3.1 Materials

The following experimental batches of device were subjected to the stability study:

Film batch P1251A0 produced with Mesoglycan batch 08-O-041
Film batch P1270C0 produced with Mesoglycan batch 08-O-023
Film batch P1271C0 produced with Mesoglycan batch 09-O-051

Theoretical content 30.10 mg of Mesoglycan.

### 3.2 Stability programme

The conditions of storage and the times at which the analyses were carried out are as follows:

**25°C** - **60% rh**
0 months, 1 month, 3 months, 6 months and 12 months
**30°C** - **65% rh**
1 month, 3 months, 6 months, 9 months and 12 months
**40°C** - **75% rh**
1 month, 3 months and 6 months

### 3.3 Method for determining the content of Mesoglycan in the biofilm evaluated through its activated anti-Xa factor activity

As known, heparin is able to inhibit activated Xa factor (FXa), through interaction with serine protease Antithrombin III (ATIII).
The method for determining anti-Xa activity, developed to determine the activity of heparin, if applied to Mesoglycan is able to indicate the activity of the product since one of its components is heparin.
The test is carried out in purified systems.
In the *in vitro* system, a sample containing known amounts of Mesoglycan and ATIII, is added to with an excess of FXa. The residual FXa is measured thanks to its amidolytic activity on the chromogenic substrate. The amounts of residual FXa is inversely proportional to the amount of Mesoglycan present in the sample.

### 3.3.1 *Reactives*

- Kit Stachrom Heparin DIAGNOSTICA STAGO cod. 00906 consisting of:

  • Buffer Tris EDTA pH 8.4 concentrated (normally dilute 10 mL to 100 mL with deionized water)
  • Chromogenic substrate CBS 31.39 (to be made up with 4 mL of deionized water)
  • Bovine ATIII (to be made up with 2 mL of diluted buffer)
  • Bovine FXa (to be made up with 4 mL of deionized water) to be prepared at least 30 minutes before use.

  - Heparin Working Standard batch 2247 that was attributed the amount 196 International anti-Xa (UIanti-Xa/mg) activity Units against International Reference Standard 3.1 at amount 1010 International anti-coagulant activity Units (UI/mL), according to the method from EP paragraph 2.7.5.
  - biofilm of dimensions 8x12 cm, theoretical weight 650 mg and theoretical content 30.10 mg of Mesoglycan
  - Sodium mesoglycan batch used for the preparation of the biofilm
  - Emulsion of reference code 9756904 Instrumentation Laboratory

### 3.3.2- *Apparatuses*

Coagulation analyzer Instrumentation Laboratory mod. ACL 300R or equivalent
Rotors code 68000 Instrumentation Laboratory
Sample cups code 67992 Instrumentation Laboratory
Scale AX 105DR fixed at 4 decimal places
Conventional laboratory glasswork and instruments

### 3.4 Preparation of the solutions for the construction of the Heparin working standard

Prepare a solution of heparin Working Standard, at the concentration of 10 mg/mL equal to 1960 UI/mL) in distilled water. From this solution prepare, by dilution with buffer Tris-EDTA at pH 8.4, other solutions at concentrations of:

> 0.15 UI/mL,
> 0.125 UI/mL,
> 0.1 UI/mL,
> 0.075 UI/mL, 0,05 UI/mL.

### 3.5 Preparation of the solutions for calculating the activity of Mesoglycan

Prepare a solution of sodium Mesoglycan, batch used for the preparation of the biofilm, at the concentration of 10 mg/mL.
Dilute with buffer Tris-EDTA to pH 8.4, said solution at the concentrations of
4 $\mu$g/mL,
3 $\mu$g/mL, 2.5 $\mu$g/mL,
2 $\mu$g/mL,
1.25 $\mu$g /mL,

### 3.5.1 *Dissolution of the film and preparation of the sample*

The film is detached from the plasticized support, it is weighed, it is cut into small pieces and it is transferred into a 600 mL beaker. 300 mL of deionized water is added and it is left under sustained agitation for about 5 minutes until completely broken up. The solution obtained is cloudy but not particularly dense (theoretical concentration of Mesoglycan: 30.10 mg/300 mL, i.e. 100.33 $\mu$g/mL).
Prepare a solution with final concentration of 3.01 $\mu$g/mL).

### 3.5.2 *Process for use of Coagulation analyzer*

In the rotors of the coagulation analyzer, according to the instructions described by the procedures, the sample cups were placed containing the reactives for each of the 5 concentrations of heparin working standard, of the 5 concentrations of Mesoglycan used as raw material for that corresponding batch of device and of the 5 samples at the same theoretical concentration of 3.01 $\mu$g/mL of biofilm.
In the reaction the heparin allosterically modifies the antithrombin III that will inhibit the Xa factor. The reaction is monitored from the absorbance detected at 405 nm.

### 3.6 Calculations to obtain the content of Mesoglycan

Based on the activity of the Heparin used (UI/mL) and on the corresponding absorbance obtained, the calibration working standard is built. From this standard, knowing the absorbance of the solutions of Mesoglycan the activity can be extrapolated in International anti-Xa Units (UaXa/mL).
In order to calculate anti-Xa activity expressed in UaXa/mg, the following formula was used:

$$\text{activity (UaXa/mg)} = \frac{a}{c} \times 1000$$

where:

a = Mesoglycan activity extrapolated from the curve expressed in UaXa/mL
c = concentration of the Mesoglycan expressed in $\mu$g/mL

The values calculated for the corresponding concentrations of Mesoglycan tested are averaged obtaining the content (T) expressed in UaXa/mg.

*Calculations to obtain the content of Mesoglycan in the biofilm*

Based on the activity of the Heparin used (UI/mL) and on the corresponding absorbance obtained, the calibration working standard is built. From this standard, knowing the absorbance of the solutions of the film the activity is extrapolated in UaXa/ml. In order to work out the concentration of Mesoglycan present in the biofilm solution expressed in $\mu$g/mL the following formula was used:

$$\text{Concentration Mesoglycan } (\mu g/mL) = \frac{a}{T} \times 1000$$

where

a = biofilm activity extrapolated from the curve expressed in UaXa/mL
T = Mesoglycan content expressed in UaXa/mg

The five values calculated, obtained in the 5 replications of the dilution of the biofilm tested, are averaged obtaining the concentration (C) of Mesoglycan in the biofilm solution expressed in $\mu$g/mL.

In order to obtain the content of Mesoglycan in the biofilm, expressed in percentage over the theoretical value of 30.10 mg, the following formula was used:

$$\text{Mesoglycan \% } = \frac{C}{3.01} \times 100$$

where:

C = concentration found (C) of Mesoglycan in the film expressed in $\mu$g/mL
3.01 theoretical concentration of Mesoglycan expressed in $\mu$g/mL

Make the average of the values obtained from the different films (at least three 3) to obtain the content of Mesoglycan in the film.

3 different water-soluble films were used, prepared as described in example 1 for each time; for batches P1270C0 and P1271C0 at $T_0$ and for batch P1251A0 at $T_{1month}$ 5 of them were used.

3.6.1 *Results*

Table 3 shows the results relative to the film P1251A0 placed in stability at 25°C and 60% relative humidity (r.h), at 30°C and 65% r.h, for 12 months and at 40° C and 75% r.h. for 6 months

Table 3. Batch pf biofilm P1251A0

| Conditions | Months | Percentage recovery of anti-Xa activity in relation to the theoretical weight of the membrane **Average $\pm$ (t $\cdot$ CV%)/(g.l.)$^{1/2}$** ooo | Mesoglycan content Batch 08-O-041 UI-anti-Xa/mg |
|---|---|---|---|
| | 0 | 86.37 $\pm$ 2.87 % | 39 |
| 25° C 60% r.h. | 1 | 85.47 $\pm$ 4.06 % | 41 |
| | 3 | 86.45 $\pm$ 5.20 % | 45 |
| | 6 | 86.45 $\pm$ 4.52 % | 46 |
| | 12 | 82.69 $\pm$ 4.28 % | 47 |
| 30° C 65% r.h. | 1 | 81,12 $\pm$ 5.39 % | 41 |
| | 3 | 90.35 $\pm$ 3.89 % | 45 |
| | 6 | 84.32 $\pm$ 2.33 % | 45 |
| | 9 | 88.09 $\pm$ 5.73 % | 41 |
| | 12 | 81.20 $\pm$ 2.29 % | 47 |
| 40° C 75% r.h. | 1 | 82.29 $\pm$ 4.17 % | 41 |
| | 3 | 87.39 $\pm$ 2.78 % | 45 |
| | 6 | 77.29 $\pm$ 1.86 % | 44 |
| | 6♠♠ | 88.88 $\pm$ 3.60 % | |
| | | | |
| Note for Table 3: the t of Students for the probability of 95% and for n -1 (15 -1) degrees of freedom is 2.15; ooo for complex methods the error of the method can be represented by **X% $\pm$ t$\cdot$ CV% / (n-1)$^{1/2}$** Where **(n-1)$^{1/2}$** for 15 measurements = **3.74** ♠♠ Replication of analysis carried out on other 3 membranes meanwhile kept at 20 °C. | | | |

In the analysis period at 1 month the mesoglycan 08-O-041 gave 41 IU/mg with CV% 3.52. In the analytical period of 12 months it gave 47 IU/mg with CV% = 6.25.

Table 4 shows the results relative to the film P1270C0 placed in stability in the conditions indicated above

Table 4. Batch of biofilm P1270CO

| Conditions | Months | Percentage recovery of anti-Xa activity in relation to the theoretical weight of the membrane **Average $\pm$ (t$\cdot$ CV%)/(g.l.)$^{1/2}$** ooo | Content of Mesoglycan Batch 08-O-023 IU anti-Xa |
|---|---|---|---|
| | 0 | 82.19 $\pm$ 3.57 % ▲ | 41 |
| 25°C 60% r.h. | 1 | 85.68 $\pm$ 4.02 % | 42 |
| | 3 | 76.99 $\pm$ 4.51 % | 44 |
| | 6 | 89.53 $\pm$ 2.36 % ** | 42 |
| | 12 | 74.64 $\pm$ 3.42 % | 45 |
| 30°C 65% r.h. | 1 | 71.66 $\pm$ 4.17 % | 42 |
| | 3 | 73.31 $\pm$ 5.85 % | 44 |
| | 6 | 81.06 $\pm$ 3.58 % ** | 42 |
| | 9 | 84.97 $\pm$ 3.46 % | 41 |
| | 12 | 83.83 $\pm$ 3.30 % | 45 |
| 40°C 75% r.h. | 1 | 79.80 $\pm$ 2,92 % | 42 |
| | 3 | 78.01 $\pm$ 2.36% | 44 |

(continued)

| Conditions | Months | Percentage recovery of anti-Xa activity in relation to the theoretical weight of the membrane<br>**Average $\pm$ (t$\cdot$ CV%)/(g.l.)$^{1/2}$ $_{ooo}$** | Content of Mesoglycan Batch 08-O-023 IU anti-Xa |
|---|---|---|---|
| | 6 | **84.12 $\pm$ 2.07%** | 42 |
| Note for Table 4: ▲ 25 measurements were carried out. For 25 measurements **(n-1)$^{1/2}$ = 4.90 and the t $_{student}$ = 2.06** (**) only 14 values. For 14 values $t_{student}$ = 2,16. For 14 values **(n-1)$^{1/2}$ = 3.60** | | | |

The batch of mesoglycan 08-O-023 in the analysis period of 9 months gave the value 41 IU/mg, (anti-Xa) with CV % 6.16. In the analytical period of 12 months it gave 45 IU/mg with CV% 6.83.

Table 5 shows the results relative to the film P1271C0 placed in stability at 25° C and 60% r.h., at 30° C and 40% r.h. for 12 months and for 6 months at 40°C and 75 r.h.

Table 5. Batch of biofilm P1271CO

| Conditions | Months | Percentage recovery of anti-Xa activity in relation to the theoretical weight of the membrane<br>**Average $\pm$ (t $\cdot$ CV%)/(g.l.)$^{1/2}$ $_{ooo}$** | Content of Mesoglycan Batch 09-O-051 UI anti-Xa/mg |
|---|---|---|---|
| | 0 | **75.21 $\pm$ 2.85 % ▲** | 39 |
| 25° C<br>60% r.h. | 1 | **78.87 $\pm$ 3.31 %** | 44 |
| | 3 | **73.05 $\pm$ 8.61 %** | 45 |
| | 6 | **82.80 $\pm$ 5.01 %** | 44 |
| | 12 | **76.04 $\pm$ 3.40 %** | 46 |
| 30° C<br>65% r.h. | 1 | **76.52 $\pm$ 3.33 %** | 44 |
| | 3 | **77.38 $\pm$ 5.75 %** | 45 |
| | 6 | **75.55 $\pm$ 3.0 %** | 44 |
| | 9 | **77.70 $\pm$ 2.82 %** | 45 |
| | 12 | **76.28 $\pm$ 2.01 %** | 46 |
| 40° C<br>75% r.h. | 1 | **73.99 $\pm$ 2.06 %** | 44 |
| | 3 | **73.76 $\pm$ 2.99 %** | 45 |
| | 6 | **80.92 $\pm$ 2.17 %** | 45 |
| Note for Table 5: ▲25 measurements were carried out. For 25 measurements **(n-1)$^{1/2}$ = 4.90** and t $_{student}$ = 2.06 | | | |

The batch of mesoglycan 09-O-051 in the analytical period of 9 months gave 45 IU/mg with CV% 7.39 and in the period of 12 months, 46 IU /mg with CV% = 5.91.

The third column of each table indicates the content of Mesoglycan in each film tested (average 5 replications carried out on 3 or on 5 different membranes)

Since the experimental weight of the three batches of film was 550 mg $\pm$ 15 mg, equal to about 85% of the theoretical one; this difference was considered in the calculation of the percentage recovery of the activity by the biofilm.

The confidence limits of a complex method can be quantified through the following formula: $\pm$ (t • CV%)/(n-1)$^{1/2}$ in which n is the number of replications carried out and t Student is calculated at 95% probability for n-1 degrees of freedom.

Since it is a fairly complex biological method and therefore subject to errors, it was decided, for each work session, to test the also active ingredient used for the preparation of each batch of water-soluble film; with the intention of checking the reliability of the results obtained in each session. The fourth column of each table shows the contents of the batch of Mesoglycan used for that batch of film. Generally, all 3 films were evaluated in a single session and therefore the content of the Mesoglycan is just one, where become necessary to carry out the analysis over many sessions the content of Mesoglycan is given for each session.

3.6.2 *Discussion*

All biofilms tested in the stability study had a water content of around 14% $\div$ 16%. Therefore the recovery of Mesoglycan from the matrix can be approximated to 90%, like in the case of batch P1271C0 or to 100% of the theoretical content, like in the case of batch P1251C0. This experimental evidence confirms the stability of the Mesoglycan in the matrix, but in particular it demonstrates the tendency of the film to release the active ingredient in the area of the lesion where it is needed.

The method has shown a certain imprecision deriving from the complexity of the analysis and from the non-parallelism between the working standards of Heparin and those of Mesoglycan. Having thus ascertained the reason for the wide confidence limits, the medical device can be considered stable over time at all of the conditions tested.

## Example 4

### Evaluation of the wound healing of the water-soluble film

The experiment consisted of evaluating the wound healing of the biofilm in reducing/closing a wound induced artificially. The experiment was carried out on a monolayer of human keratinocyte cells (hacat); on which a scrape was created by running a blade along the entire monolayer. The healing and regenerative efficiency of the device at the concentrations of 5 mg/mL, 2.5 mg/mL and 1.25 mg/mL of water-soluble film was determined by taking photographs of the cell mediums at times $T_0$, $T_{2h}$, $T_{4h}$, $T_{8h}$ and $T_{24h}$ in comparison with the same cell medium not treated. Experimental evidence was obtained that the edges of the wound spontaneously started to heal in the control, but at the concentrations of 5 mg/mL, 2.5 mg/mL and 1.25 mg/mL of film, already after 2 hours, 4 hours and 8 hours the healing rate is greater with respect to the rate of spontaneous healing detected in the controls. The variations in size of the wound, understood as the greater healing rate, in the treated mediums with respect to the control medium are significant at times $T_{2h}$ and $T_{4h}$. The water-soluble film increases its cell regeneration properties, stimulating their proliferation and migration thus making their healing capability faster and more effective.

## Example 5

### Modulation of the buffering effect of the film.

### Example 5. Modulation of the buffering effect of the film.

A sample of water-soluble film was prepared according to the recipe of example 1, with the difference that in the preparation mixture at the moment of the control of the pH a suitable amount of the organic hydroxy acid citric acid was added, until a pH of the system was obtained equal to pH 5.6 (0.25 g of citric acid were added to 100 g of biofilm mix containing 76% water. Therefore, the added citric acid represented 1.04 % by weight over the total dry weight of the components).. In the preparation described in example 1, the pH was at this point 7.0. The water-soluble film obtained, subjected to the evaluation model as described in example 2, demonstrated that it managed to maintain the variable pH values only in the pH range 6.0 $\pm$ 0.4, although with the additions of hydrochloric acid or sodium hydroxide 0.01M, in the respective solutions and at the quantities described in the experiment of example 2.

Table 6a shows the pH values of the aqueous solutions each prepared by solubilization in 20 ml of water, of a membrane of biofilm, containing a known amount of an organic hydroxy acid, (citric acid) in addition to the mixture given in example 1 and inspired by US2010/0029790.

**Table 6a**

| | pH values of membranes prepared according to example 5, solubilized in 20 ml of water and made basic (NaOH 0,01 M) and acidic (HCl 0,01M) with the addition of the amounts indicated at the side. | | | |
|---|---|---|---|---|
| NaOH 0,01M added (μL) | Membrane in 20 mL of water + NaOH 0,01 M | | Membrane in 20 mL of water + HCl 0,01 M | HCl 0,01 M added (μL) |
| _ | 5.9 | | 5.9 | - |
| 20 | 5.9 | | 5.9 | 20 |
| 100 | 5.95 | | 5.9 | 100 |
| 200 | 6.0 | | 5.8 | 200 |
| 400 | 6.1 | | 5.8 | 400 |
| 1000 | 6.35 | | 5.6 | 1000 |

The amount of variability of the pH of such a preparation is included in the range 5,9 ± 0,5. Therefore, it also includes pH 6,0, the optimal result for a significant advantage in the healing speed of varicose ulcers, as shown in VASA 1979; 8 (4): 339-349, on page 14 of the present application, from line 24 onwards.

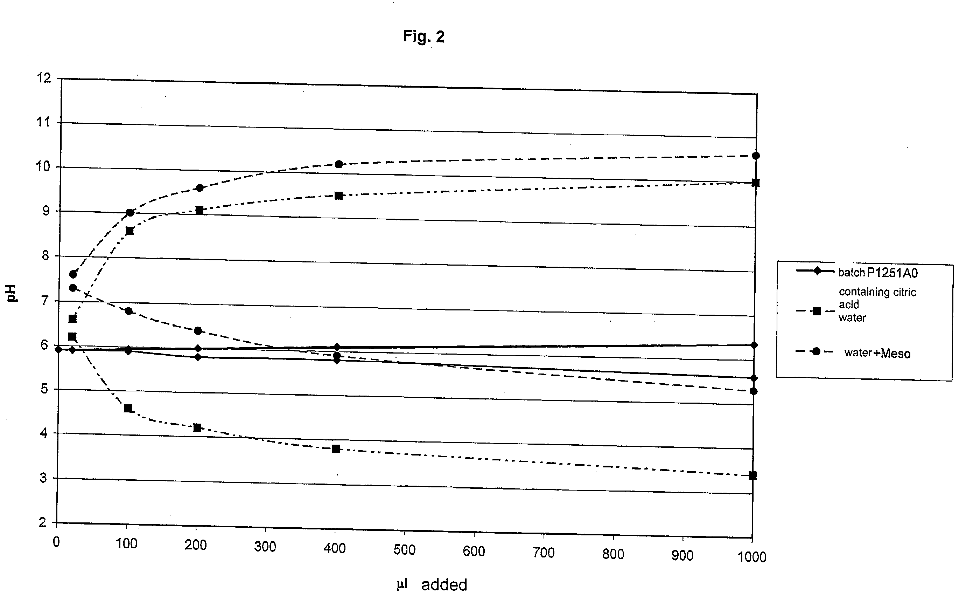

**Figure 2 gives the results of the buffering effect of the biofilm prepared as described in example 5.**

Figure 2 shows the progression of the pHs of said matrix placed in solutions made acidic with HCl and basic with NaOH 0,01 M ♦, and the comparison of the pH values in aqueous solutions ■, and in aqueous solutions containing 30 mg of Mesoglycan ●, after the addition of the corresponding volumes of NaOH and HCl.

This buffering characteristic of the film was obtained through addition of a known amount of organic hydroxy acid to the preparation mixture. In practice, by changing the quality and quantity of this addition, it is possible to give the matrix of the biofilm the ability to keep lesions and sores "buffered" in the pH environments suitable for accelerating their healing.

**Claims**

1.    Water-soluble film containing

a) between 25 and 60% by weight over the total weight of the film of sodium alginate as main filmogenous substance;
b) between 0.1 and 20% by weight of microcrystalline cellulose over the total weight of the film,
c) between 0.1 and 25% by weight of vegetable proteins ,
d) at least one glycosaminoglycan sulphate and/or galactosaminoglycan sulphate, as active ingredient with healing activity.

2.    Water-soluble film according to claim 1, **characterised in that** said glycosaminoglycan sulphate and/or galactosaminoglycan sulphate is selected from the class consisting of: chondroitin sulphate, dermatan sulphate, heparan sulphate, heparin, keratan sulfate and relative mixtures of at least two of the aforementioned components.

3.    Water-soluble film according to claim 2, **characterised in that** said heparin has an average molecular weight comprised between 4000 and 6500 and with 60% of the molecules having weights less than 8000 Da.

4.    Water-soluble film according to any one of claims 1-2, **characterised in that** the heparin has an average molecular weight comprised between: 6500 and 10500 and with at least 60% of the molecules **characterised by** an average molecular weight of less than 12000 Da.

5.    Water-soluble film according to any one of claims 1-2, **characterised in that** the heparin has an average molecular weight comprised between 12000 and 18000 Da.

6.    Water-soluble film according to any one of claims 1, 2, 4, **characterised in that** the glycosaminoglycan sulphate and/or galactosaminoglycan sulphate consists of a mixture (A) comprising:

α) heparin with molecular weight 6500 and 12000 and with at least 60% of the molecules **characterised by** an average molecular weight of less than 12000 Da.
β) dermatan sulphate
γ) chondroitin sulphate.

7.    Water-soluble film according to claim 6, **characterised in that** the mixture (A) consists of:

• 55-60% a mixture of heparin with average molecular weight comprised between 6500 and 10500 Da
• 25-35% dermatan sulphate,
• 4-8% chondroitin sulphate (mostly Ch-S-A)

8.    Water-soluble film according to any one of claims 1-2, wherein the glycosaminoglycan sulphate and/or galactosaminoglycan sulphate consists of the mixture (B) consisting of:

α') heparin with relatively low molecular weight,
β') chondroitin sulphate.

9. Water-soluble film according to claim 8, **characterised in that** the mixture (B) consists of 80% heparin with relatively low molecular weight and 20% dermatan sulphate.

10. Water-soluble film according to any one of claims 1-2, wherein glycosaminoglycan sulphate and/or galactosaminoglycan sulphate is a mixture (C) consisting of:

   $\alpha$") heparan sulphate,
   $\beta$") chondroitin sulphate
   $\gamma$") dermatan sulphate.

11. Water-soluble film according to any one of claims 1-5, wherein the glycosaminoglycan and/or galactosaminoglycan sulphate is a mixture (D) consisting of:

   $\alpha$"') dermatan sulphate;
   $\beta$"') chondroitin sulphate.

12. Water-soluble film according to claim 10, wherein said mixture (C) consists of 75-85% heparan sulphate, no more than 8.5% by weight chondroitin sulphates (Ch-S-A) and (Ch-S-C), and between 8 and 16% dermatan sulphate.

13. Water-soluble film according to claim 6, wherein the mixture (A) is contained in the water-soluble film in concentrations comprised between 0.1 and 10% by weight calculated over the dry weight of said film.

14. Water-soluble film according to any one of claims 1-13 comprising an organic hydroxy acid.

15. Water-soluble film according to claim 14 wherein said hydroxy acid has between 3 and 6 carbon atoms and between 1 and 2 hydroxyl groups.

16. Water-soluble film according to any one of claims 14 and 15, **characterised in that** this organic hydroxy acid is selected from: citric acid, tartaric acid, tartronic acid, hydroxybutyric acid, etc.

17. Water-soluble film according to any one of claims 14-16, wherein said organic hydroxy acid is added to the mixture in quantities such that the mixture reaches a pH comprised between 5.4 and 5.6.

18. Device for medical use comprising the water-soluble film according to any one of claims 1-17.

19. Device for medical use according to claim 18, **characterised in that** it consists of the water-soluble film according to any one of claims 1-17.

20. Device for medical use according to any one of claims 18 or 19, for the treatment of:

   • secondary lesions following to pathological events, selected fom ulcers of the lower limbs in subjects with chronic venous insufficiency, diabetic foot ulcer, arterial ulcers,
   • secondary lesions following to traumatic events selected from bedsores, abrasions,
   • secondary lesions following to environmental events selected from lesions from scalding , bums.

**Patentansprüche**

1. Wasserlöslicher Film, der folgendes enthält:

   a) zwischen 25 und 60% im Gewicht bezogen auf das Gesamtgewicht des Filmes aus Natriumalginat als Hauptfilmbildender Stoff;
   b) zwischen 0,1 und 20% im Gewicht aus mikrokristalliner Cellulose bezogen auf das Gesamtgewicht des Filmes,
   c) zwischen 0,1 und 25 % im Gewicht aus pflanzlichen Proteinen,
   d) mindestens ein Glykosaminoglykansulfat und/oder Galactosaminoglycansulfat als Wirkstoff mit Heiltätigkeit.

2. Wasserlöslicher Film nach Anspruch 1, **dadurch gekennzeichnet, dass** das genannte Glykosaminoglykansulfat und/oder Galactosaminoglycansulfat aus der Klasse ausgewählt wird, die aus Chondroitinsulfat, Dermatansulfat, Heparansulfat, Heparin, Keratansulfat und relativen Mischungen von mindestens zwei der oben genannten Komponenten besteht.

3. Wasserlöslicher Film nach Anspruch 2, **dadurch gekennzeichnet, dass** Heparin einen durchschnittlichen Molekulargewicht zwischen 4000 und 6500 Da hat und dass 60% der Moleküle einen Gewicht von weniger als 8000 Da haben.

4. Wasserlöslicher Film nach einem der Ansprüche 1-2, **dadurch gekennzeichnet, dass** Heparin einen durchschnittlichen Molekulargewicht zwischen 6500 und 10500 Da hat und mit mindestens 60% der Moleküle, die von einem durchschnittlichen Molekulargewicht von weniger als 12000 Da gekennzeichnet sind.

5. Wasserlöslicher Film nach einem der Ansprüche 1-2, **dadurch gekennzeichnet, dass** Heparin einen durchschnittlichen Molekulargewicht zwischen 12000 und 18000 Da hat.

6. Wasserlöslicher Film nach einem der Ansprüche 1, 2, 4, **dadurch gekennzeichnet, dass** das Glykosaminoglykansulfat und/oder Galactosaminoglycansulfat aus einer Mischung (A) besteht, die folgendes umfasst:

   $\alpha$) Heparin mit einem molekularen Gewicht von 6500 und 12000 Da und mit mindestens 60% der Moleküle, die von einem durchschnittlichen Molekulargewicht von weniger als 12000 Da gekennzeichnet sind,
   $\beta$) Dermatansulfat,
   $\gamma$) Chondroitinsulfat.

7. Wasserlöslicher Filme nach Anspruch 6, **dadurch gekennzeichnet, dass** die Mischung (A) aus folgendem besteht:

   • 55-60% einer Mischung aus Heparin mit durchschnittlichem Molekulargewicht zwischen 6500 und 10500 Da,
   • 25-35% Dermatansulfat,
   • 4-8% Chondroitinsulfat (meist Ch-S-A).

8. Wasserlöslicher Folie nach einem der Ansprüche 1-2, **dadurch gekennzeichnet, dass** Glykosaminoglykansulfat und/oder Galactosaminoglycansulfat aus der Mischung (B) besteht, die aus folgende besteht:

   $\alpha$') Heparin mit relativ niedrigem Molekulargewicht,
   $\beta$') Chondroitinsulfat.

9. Wasserlöslicher Film nach Anspruch 8, **dadurch gekennzeichnet, dass** die Mischung (B) aus 80% Heparin mit relativ niedrigem Molekulargewicht und 20% Dermatansulfat besteht.

10. Wasserlöslicher Film nach einem der Ansprüche 1-2, **dadurch gekennzeichnet, dass** Glykosaminoglykansulfat und/oder Galactosaminoglycansulfat eine Mischung (C) sind, die aus folgendem besteht:

    $\alpha$") Heparansulfat,
    $\beta$") Chondroitinsulfat,
    $\gamma$") Dermatansulfat.

11. Wasserlöslicher Film nach einem der Ansprüche 1-5, **dadurch gekennzeichnet, dass** Glykosaminoglykansulfat und/oder Galactosaminoglycansulfat eine Mischung (D) sind, die aus folgendem besteht:

    $\alpha$'") Dermatansulfat,
    $\beta$'") Chondroitinsulfat.

12. Wasserlöslicher Film nach Anspruch 10, **dadurch gekennzeichnet, dass** die obengenannte Mischung (C) aus 75-85% im Gewicht Heparansulfat, nicht mehr als 8,5% im Gewicht Chondroitinsulfat (Ch-S-A) und (Ch-S-C) und zwischen 8 und 16% im Gewicht Dermatansulfat besteht.

13. Wasserlöslicher Film nach Anspruch 6, **dadurch gekennzeichnet, dass** die Mischung (A) in dem wasserlöslichen Film in Konzentrationen enthalten ist, die zwischen 0,1 und 10% im Gewicht, bezogen auf das Trockengewicht des Filmes, sind.

**14.** Wasserlöslicher Film nach einem der Ansprüche 1-13 umfassend organischen Hydroxysäure .

**15.** Wasserlöslicher Film nach Anspruch 14, wobei die obengenannte Hydroxysäure zwischen 3 und 6 Kohlenstoffatomen und zwischen 1 und 2 Hydroxylgruppen enthält.

**16.** Wasserlöslicher Film nach einem der Ansprüche 14 und 15, **dadurch gekennzeichnet, dass** diese organische Hydroxysäure unter Zitronensäure, Weinsäure, Tartronsäure, Hydroxybuttersäure, etc. ausgewählt wird.

**17.** Wasserlöslicher Film nach einem der Ansprüche 14 bis 16, wobei die obengenannte organische Hydroxysäure zur Mischung in Mengen hinzugefügt wird, bei denen die Mischung einen pH-Wert zwischen 5,4 und 5,6 erreicht.

**18.** Vorrichtung zur medizinischen Verwendung, die den wasserlöslichen Film nach einem der Ansprüche 1 bis 17 umfasst.

**19.** Vorrichtung zur medizinischen Verwendung nach Anspruch 18, **dadurch gekennzeichnet, dass** sie aus dem wasserlöslichen Film nach einem der Ansprüche 1 bis 17 besteht.

**20.** Vorrichtung zur medizinischen Verwendung nach einem der Ansprüche 18 oder 19 zur Behandlung von:

- sekundären Läsionen nach pathologischen Ereignissen, ausgewählt aus Geschwüren der unteren Gliedmaßen bei Patienten mit chronischer venösen Insuffizienz, diabetischem Fußgeschwür, arteriellen Geschwüren,
- sekundären Läsionen nach traumatischen Ereignissen, ausgewählt aus Wundliegen, Abrieb,
- sekundären Läsionen nach Umweltzuständen, ausgewählt aus Läsionen von Verbrühungen, Verbrennungen.

## Revendications

**1.** Film hydrosoluble contenant

A) entre 25 et 60% en poids sur le poids total du film d'alginate de sodium en tant que principale substance filmogène ;
B) entre 0,1 et 20% en poids de la cellulose microcristalline sur le poids total du film,
C) entre 0,1 et 25% en poids de protéines végétales,
D) au moins un sulfate de glycosaminoglycane et/ou un sulfate de galactosaminoglycane en tant que substance active à l'activité de guérison.

**2.** Film hydrosoluble selon la revendication 1, **caractérisé en ce que** ledit sulfate de glycosaminoglycane et/ou sulfate de galactosaminoglycane est sélectionné dans la classe composée de : sulfate de chondroïtine, sulfate de dermatane, sulfate de héparane, héparine, sulfate de kératane et des relatives mélanges d'au moins deux des composants susmentionnés.

**3.** Film hydrosoluble selon la revendication 2, **caractérisé en ce que** ladite héparine a une masse moléculaire moyenne comprise entre 4000 et 6500 et avec 60% des molécules ayant des poids inférieurs à 8000 Da.

**4.** Film hydrosoluble selon l'une quelconque des revendications 1 à 2, **caractérisé en ce que** l'héparine a une masse moléculaire moyenne comprise entre 6500 et 10500 et avec au moins 60% des molécules **caractérisées par** un poids moléculaire moyen inférieur à 12000 Da.

**5.** Film hydrosoluble selon l'une quelconque des revendications 1 à 2, **caractérisé en ce que** l'héparine a une masse moléculaire moyenne comprise entre 12000 et 18000 Da.

**6.** Film hydrosoluble selon l'une quelconque des revendications 1, 2, 4, **caractérisé en ce que** le sulfate de glycosaminoglycane et/ou le sulfate de galactosaminoglycane sont constitués d'un mélange (A) comprenant :

α) héparine avec un poids moléculaire 6500 et 12000 et avec au moins 60% des molécules **caractérisées par** un poids moléculaire moyen inférieur à 12000 Da,
β) sulfate de dermatane,
γ) sulfate de chondroïtine.

**7.** Film hydrosoluble selon la revendication 6, **caractérisé en ce que** le mélange (A) consiste en :

• 55 à 60% d'un mélange d'héparine ayant une masse moléculaire moyenne comprise entre 6500 et 10500 Da,
• 25-35% de sulfate de dermatane,
• 4-8% de sulfate de chondroïtine (principalement Ch-S-A).

**8.** Film hydrosoluble selon l'une quelconque des revendications 1 à 2, où le sulfate de glycosaminoglycane et/ou le sulfate de galactosaminoglycane consiste en le mélange (B) formé par :

α') héparine avec un poids moléculaire relativement faible,
β') sulfate de chondroïtine.

**9.** Film hydrosoluble selon la revendication 8, **caractérisé en ce que** le mélange (B) consiste en 80% d'héparine avec un poids moléculaire relativement faible et 20% de sulfate de dermatane.

**10.** Film hydrosoluble selon l'une quelconque des revendications 1 à 2, où le sulfate de glycosaminoglycane et/ou le sulfate de galactosaminoglycane est un mélange (C) consistant en :

α") sulfate d'héparane,
β") sulfate de chondroïtine,
γ") sulfate de dermatane.

**11.** Film hydrosoluble selon l'une quelconque des revendications 1 à 5, où le sulfate de glycosaminoglycane et/ou le sulfate de galactosaminoglycane est un mélange (D) consistant en :

α"') sulfate de dermatane ;
β'") sulfate de chondroïtine.

**12.** Film hydrosoluble selon la revendication 10, où ledit mélange (C) consiste en 75-85% de sulfate d'héparane, pas plus de 8,5% en poids de sulfate de chondroïtine (Ch-SA) et (Ch-S-C) et entre 8 et 16% de sulfate de dermatane.

**13.** Film hydrosoluble selon la revendication 6, où le mélange (A) est contenu dans le film hydrosoluble en des concentrations comprises entre 0,1 et 10% en poids calculées sur le poids sec dudit film.

**14.** Film hydrosoluble selon l'une quelconque des revendications 1 à 13 comprenant un hydroxyacide organique.

**15.** Film hydrosoluble selon la revendication 14, où ledit hydroxyacide a entre 3 et 6 atomes de carbone et entre 1 et 2 groupes hydroxyle.

**16.** Film hydrosoluble selon l'une quelconque des revendications 14 et 15, **caractérisé en ce que** ce hydroxyacide organique est choisi parmi : acide citrique, acide tartrique, acide tartronique, acide hydroxybutyrique, etc.

**17.** Film hydrosoluble selon l'une quelconque des revendications 14 à 16, où ledit hydroxyacide organique est ajouté au mélange en quantités telles que le mélange atteint un pH compris entre 5,4 et 5,6.

**18.** Dispositif à usage médical comprenant le film hydrosoluble selon l'une quelconque des revendications 1 à 17.

**19.** Dispositif à usage médical selon la revendication 18, **caractérisé en ce qu'**il consiste en un film hydrosoluble selon l'une quelconque des revendications 1 à 17.

**20.** Dispositif à usage médical selon l'une quelconque des revendications 18 ou 19 pour le traitement de :

• lésions secondaires suite à des événements pathologiques, choisis parmi les ulcères des membres inférieurs chez les sujets atteints d'insuffisance veineuse chronique, l'ulcère du pied diabétique, les ulcères artériels,
• lésions secondaires suite à des événements traumatiques choisis parmi les escarres, les abrasions,
• lésions secondaires suite à des événements environnementaux choisis parmi les lésions causées par l'échaudage, les brûlures.

Fig. 1

EP 2 874 614 B1

Fig. 2

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 4837024 A
- US 5929050 A
- WO 03034993 A
- US 2010002790 A
- US 20100029790 A

## Non-patent literature cited in the description

- **ELNIUS K. et al.** *J. Cell. Biol.,* 1991, vol. 114, 585-595
- **GALLO et al.** *J. Invest. Dermatol.,* 1996, vol. 107, 676-683
- **PENC et al.** *J. Biol. Chem.,* 1998, vol. 273 (43), 28116-28121
- **PENC SF et al.** *J. Clin. Invest.,* 1999, vol. 103 (9), 1329-1335
- Chemistry of Scarring. **GARG et al.** Scarless Wound Repair. Marcel Dekker, 2000, 1-2
- **TRAVERSA B. et al.** *Primary Intention,* 2001, vol. 9 (4), 161-167
- **RUOZI B. et al.** *Int J. Pharmaceutics,* 2009, vol. 738 (1-2), 108-115
- Glycosamin hydrogel films as biointeractive dressings for wound healing. **KIRKER et al.** Biomaterials. Elsevier Science Publisher BV, 01 September 2002, vol. 23, 3661-3671
- **AROSIO et al.** *EUROPEAN JOURNAL OF VASCULAR AND ENDOVASCULAR SURGERY : THE OFFICIAL JOURNAL OF THE EUROPEAN SOCIETY FOR VASCULAR SURGERY OCT 2001,* October 2001, vol. 22 (4), ISSN 1078-5884, 365-372
- **GREENER B et al.** *J of Wound Care,* 2005, vol. 14 (2), 59-61
- **RUSHTON I.** Role of proteases and of pH in wound healing. *Nurs Stand,* 2007, vol. 21 (32), 68, , 70, , 72
- **V.K. SHUKLA et al.** *J of Wound Care,* 2007, vol. 16 (7), 291-294
- **IAN A.I. WILSON et al.** *VASA,* 1979, vol. 8 (4), 339-342, www.scirus.com
- **ALEXANDER L et al.** the monograph reported. *Arch Dermatol Res,* 2007, vol. 298, 413-420
- *VASA,* 1979, vol. 8 (4), 339-349